# EUROPEAN PATENT APPLICATION

(11) **EP 4 840 403 A1**
(43) Date of publication of application: **18.11.2026**
(21) Application number: 26177829.4
(22) Date of filing: 11.05.2026
(51) Int. Cl.: A61B 8/08, A61B 8/00, A61B 8/12

(54) **IMAGE PROCESSING APPARATUS, CONTROL METHOD OF IMAGE PROCESSING APPARATUS AND COMPUTER PROGRAM**

(30) Priority: 13.05.2025 JP 2025080228; 20.02.2026 JP 2026025934
(71) Applicant: Canon Kabushiki Kaisha, Tokyo 146-8501 (JP)
(72) Inventor: OTOMARU, Itaru, Ohta-ku, Tokyo, 146-8501 (JP); AMAGI, Seina, Ohta-ku, Tokyo, 146-8501 (JP)
(74) Representative: TBK

(57) **Abstract**

An image processing apparatus includes acquisition means configured to acquire an image that represents a part of a subject and is captured using a probe, and generation means configured to generate, on the basis of the image, guidance information for guiding the probe such that a predetermined structure portion of the subject located outside of an imaging range of the image is included within the imaging range.

## Description

### TECHNICAL FIELD

The present disclosure relates to an image processing apparatus, a control method of an image processing apparatus, and a computer program.

### BACKGROUND

In diagnosis using an ultrasonic diagnostic apparatus, various types of diagnosis may be performed by specifying a standard cross section of a subject and measuring anatomical features on the cross section. For example, in fetal diagnosis using an ultrasonic diagnostic apparatus, standard cross sections of three types of regions, namely, the head, abdomen, and femur of the fetus, are obtained, and features of the respective regions, such as biparietal diameter, are measured in each sectional image, thereby estimating the weight of the fetus.

In such a diagnosis, since a fetus in or after the middle stage of pregnancy is larger than the range that can be imaged in a single scan by a probe, the physician (user) who performs the diagnosis needs to search for the standard cross sections while moving the probe, to specify all of the above standard cross sections. Thus, to reduce the burden involved in the specification of standard cross sections by a physician, Japanese Translation of PCT Application No. 2021-530334 proposes a technique for guiding (navigating) the probe. Also, Standard Plane Detection in 3D Fetal Ultrasound Using an Iterative Transformation Network by Li et al., presented at the International Conference on Medical Image Computing and Computer-Assisted Intervention in 2018 (hereinafter referred to as "Non-Patent Literature 1"), proposes a method for estimating a standard cross section.

### SUMMARY

However, when a standard cross section is located outside of the imaging range, the above conventional technique may fail to appropriately guide an imaging apparatus such as a probe.

The technology of the present disclosure provides a user with suitable probe guidance information even when a predetermined structure portion of a subject is located outside of the imaging range of an image.

According to some embodiments, an image processing apparatus as specified in claim 1 is provided. Optional features are specified in claims 2 to 20. According to some embodiments, a control method is provided as specified in claim 21. According to some embodiments, a computer program is provided as specified in claim 22.

According to the technology of the present disclosure, it is possible to provide the user with suitable probe guidance information even when a predetermined structure portion of a subject is located outside of the imaging range of an image.

Features of the present disclosure will become apparent from the following description of embodiments with reference to the attached drawings. The following description of embodiments is described by way of example.

### BRIEF DESCRIPTION OF THE DRAWINGS

FIG. 1 is a diagram schematically illustrating the configuration of an image processing system according to one embodiment.
FIG. 2 is a flowchart of processing performed by an image processing apparatus according to one embodiment.
FIGS. 3A and 3B are diagrams illustrating examples of images acquired by an image processing apparatus according to one embodiment.
FIGS. 4A to 4C are diagrams illustrating examples of standard cross sections estimated by an image processing apparatus according to one embodiment.
FIG. 5 is a diagram illustrating an example of guidance information displayed by an image processing apparatus according to one embodiment.
FIG. 6 is a flowchart of the processing of obtaining a trained model in one embodiment.
FIG. 7 is a diagram schematically illustrating an example of training images in one embodiment.
FIG. 8 is a diagram schematically illustrating stitching of training images in one embodiment.
FIG. 9 is a diagram illustrating an example of a state of precise estimation displayed by an image processing apparatus in one embodiment.

### DESCRIPTION OF THE EMBODIMENTS

Hereinafter, embodiments of an image processing apparatus according to the present disclosure will be described in detail by way of example with reference to the accompanying drawings. However, it is to be understood that constituent elements described in the following embodiments are merely exemplary and that the technical scope of the present disclosure is not intended to be limited by any of the individual embodiments described below but to be defined by the aspects.

### First Embodiment

An image processing apparatus according to a first embodiment of the present disclosure is now described. The image processing apparatus of the present embodiment is an apparatus for estimating the position and orientation of three types of standard cross sections (head, abdomen, and femur) from a three-dimensional volume image of a fetus captured by an ultrasonic diagnostic apparatus including a three-dimensional abdominal probe. When it is estimated that the position of a standard cross section is outside of the range of the captured three-dimensional volume, the ultrasonic diagnostic apparatus outputs guidance information (navigation information) for prompting the user to change the probe position. Also, the ultrasonic diagnostic apparatus estimates standard cross section parameters when it is determined that the position of the standard cross section is in the three-dimensional volume.

In one example, the image processing apparatus according to the present embodiment is directed to an image of a three-dimensional volume obtained by imaging a fetus in or after the middle stage of pregnancy using a three-dimensional abdominal probe. When imaging a fetus in or after the middle stage of pregnancy with a three-dimensional abdominal probe, the fetus may be larger than the range that can be imaged in a single scan with the three-dimensional abdominal probe. Thus, when the head of a fetus is imaged with a probe, for example, the femur of the fetus may be outside of the imaging range of the probe. As such, it is assumed that an image obtained by imaging a fetus according to the present embodiment is an image in which a part of the fetus is located outside of the three-dimensional volume.

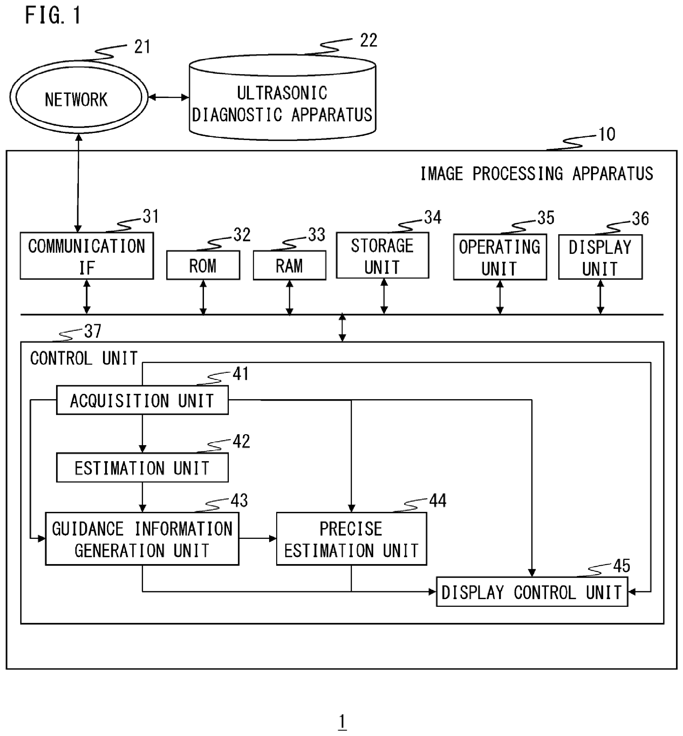

The configuration and processing of the image processing apparatus according to the present embodiment are described below with reference to FIG. 1. FIG. 1 is a block diagram illustrating an example of the configuration of an imaging system (also referred to as a medical image processing system) including an image processing apparatus of the present embodiment. The imaging system 1 according to the present embodiment includes an image processing apparatus 10 and an ultrasonic diagnostic apparatus 22.

The image processing apparatus 10 is connected to the ultrasonic diagnostic apparatus 22 via a network 21 in a communicable state. The network 21 includes, for example, a local area network (LAN) or a wide area network (WAN).

The ultrasonic diagnostic apparatus 22 images a subject using a three-dimensional probe, and generates a captured image of the three-dimensional volume of the subject. The image processing apparatus 10 includes a communication interface (IF) 31 (communication unit), a read only memory (ROM) 32, and a random access memory (RAM) 33. The image processing apparatus 10 further includes a storage unit 34, an operating unit 35, a display unit 36, and a control unit 37. Note that at least a part of the functions of the image processing apparatus 10 may be incorporated into the ultrasonic diagnostic apparatus 22.

The communication IF 31 (communication unit) is constituted by a LAN card or the like, and provides communication between an external apparatus (for example, the ultrasonic diagnostic apparatus 22 or the like) and the image processing apparatus 10. The ROM 32 is constituted by a nonvolatile memory or the like and stores various programs and various data for performing processing described below. The RAM 33 is constituted by a volatile memory or the like, and is used as a work memory for temporarily storing a program and data for processing that is being performed by the image processing apparatus 10. The storage unit 34 is constituted by a hard disk drive (HDD) or the like, and stores various programs and data used in the image processing apparatus 10. The operating unit 35 is composed of a keyboard, a mouse, a touch panel, or the like, and receives input of instructions from a user (such as a physician or a medical technician). The display unit 36 is constituted by a display or the like, and displays to the user various types of information generated by processing described below.

The control unit 37 is constituted by a central processing unit (CPU) or a dedicated or general-purpose processor. The control unit 37 may be constituted by a graphic processing unit (GPU) or a field-programmable gate array (FPGA). Also, the control unit 37 may be formed by an application specific integrated circuit (ASIC) or the like. The control unit 37 includes an acquisition unit 41, an estimation unit 42, a guidance information generation unit 43, a precise estimation unit 44, and a display control unit 45, which are described below.

The acquisition unit 41 acquires, from the ultrasonic diagnostic apparatus 22, an image of a three-dimensional volume obtained by imaging at least a part of a fetus as a subject. That is, the acquisition unit 41 acquires an image representing a part of the subject captured using a probe.

Based on the three-dimensional volume image acquired by the acquisition unit 41, the estimation unit 42 estimates the position and orientation of a plurality of standard cross sections including a standard cross section located outside of the imaging range of the three-dimensional volume.

Based on the position and orientation of the standard cross sections estimated by the estimation unit 42, the guidance information generation unit 43 determines whether there is a standard cross section on which precise estimation can be performed. When it is determined that there is no standard cross section on which precise estimation can be performed, the guidance information generation unit 43 generates guidance information to assist the user in operating the probe. The guidance information generation unit 43 acquires information about the position, such as the center position, of a predetermined structure portion (for example, a standard cross section) estimated by the estimation unit 42, and generates guidance information on the basis of the information. In other words, on the basis of the image acquired by the acquisition unit 41, the guidance information generation unit 43 generates guidance information for guiding the probe such that a predetermined structure portion of the subject located outside of the imaging range of the image is included in the imaging range.

The precise estimation unit 44 estimates parameters representing the position and orientation of a standard cross section on which precise estimation is determined to be executable by the guidance information generation unit 43.

Based on the guidance information generated by the guidance information generation unit 43, the parameters estimated by the precise estimation unit 44, and the guidance information, the display control unit 45 performs control for displaying an image necessary for the user to move the probe and observe the subject.

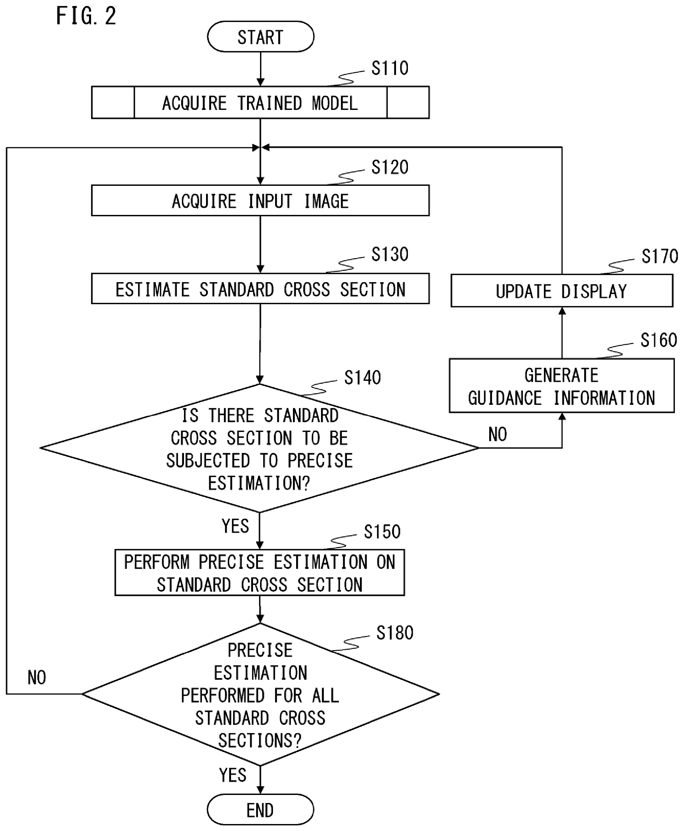

Referring to the flowchart of FIG. 2, an example of processing performed by the image processing apparatus 10 in the present embodiment is now described.

(Step S110: Acquire trained model) At step S110, the estimation unit 42 acquires a trained model that estimates the position and orientation of a plurality of standard cross sections including a standard cross section located outside of the imaging range of a three-dimensional volume from an image of the three-dimensional volume. The estimation unit 42 then stores the acquired trained model in the RAM 33.

In this embodiment, the plurality of standard cross sections include three types of standard cross sections, namely, a standard cross section of the head of the fetus, a standard cross section of the abdomen, and a standard cross section of the femur. Also, the trained model acquired at this step is a trained model that has been trained so as to estimate estimated values of parameters representing the position and orientation of these three types of standard cross sections when an image of a three-dimensional volume is input. For example, when an image obtained by capturing only the head region of a fetus as an image of a three-dimensional volume is input to the trained model, the estimation results of the position and orientation of standard cross sections of the abdomen and femur of the fetus located outside of the image are output from the trained model.

Also, in this embodiment, it is assumed that the position and orientation of a standard cross section are given by parameters represented by a total of seven numerical values including three parameters of center position coordinates of the standard cross section, three parameters of a rotation axis vector of the standard cross section, and one parameter of a rotation angle of the standard cross section. The present embodiment estimates three types of standard cross sections, so that the position and orientation of these three types of standard cross sections are represented by a total of 21 parameters. The estimated "center position of the standard cross section" is the relative position of the input image from the center position of the three-dimensional volume, and the estimated "orientation of the standard cross section" is the relative orientation of the input image in the image coordinate system of the three-dimensional volume.

The trained model used in the present embodiment may be a model that is constructed in advance and acquired from a database (not illustrated) or the storage unit 34, or may be constructed by performing learning processing in the processing of this step. Alternatively, the image processing apparatus 10 may acquire a trained model constructed by performing training using a computer device different from the image processing apparatus 10 via the network 21.

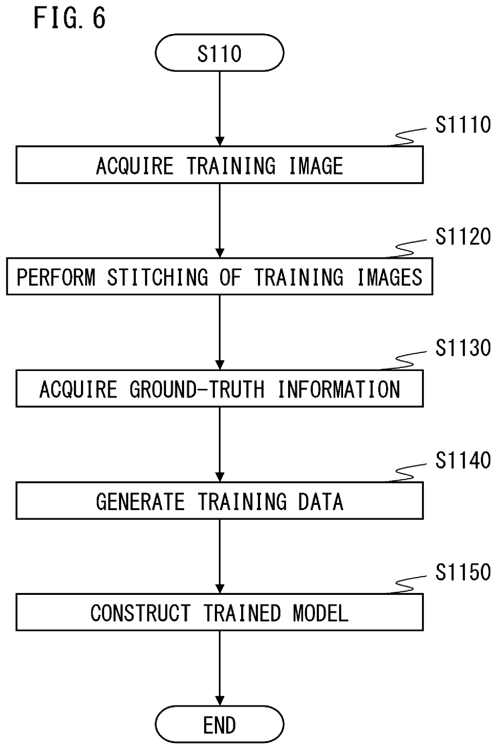

An example of specific construction processing of the trained model acquired at this step will be described below using the flowchart of steps S1110 to S1150 illustrated in FIG. 6.

(Step S120: Acquire input image) At step S120, the acquisition unit 41 acquires from the ultrasonic diagnostic apparatus 22 a three-dimensional volume image capturing at least a partial region of a fetus, which is the subject. The acquisition unit 41 then stores the acquired three-dimensional volume image in the RAM 33.

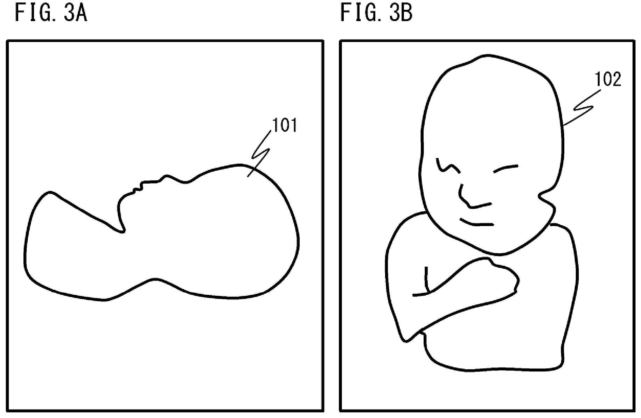

Referring to FIGS. 3A and 3B, an example of a three-dimensional volume image representing a part of a subject acquired at this step is now described. FIG. 3A illustrates an example of a sagittal sectional image 101 of a three-dimensional image of a fetus generated from an image captured by a current probe. FIG. 3B illustrates a volume rendering image 102 of a three-dimensional image of a fetus as an example of a three-dimensional volume image acquired at this step.

(Step S130: Estimate standard cross section) At step S130, the estimation unit 42 inputs the three-dimensional volume image acquired at step S120 to the trained model acquired at step S110, and estimates the position and orientation of a plurality of standard cross sections. The estimation unit 42 stores parameters of each standard cross section acquired by the estimation in the RAM 33.

The type and representation format of the standard cross sections are as described in step S110. In other words, a total of 21 parameters represent the three types of standard cross sections, which are estimation targets. However, when this step is performed again after step S150, which will be described below, the control unit 37 assigns, to a standard cross section for which precise estimation has already been performed at step S150, an identifier indicating that estimation of the standard cross section has been completed, for example, an identifier "finalized". Then, the control unit 37 excludes the standard cross section with the "finalized" identifier from the targets for the estimation processing at the present step and the processing at subsequent steps.

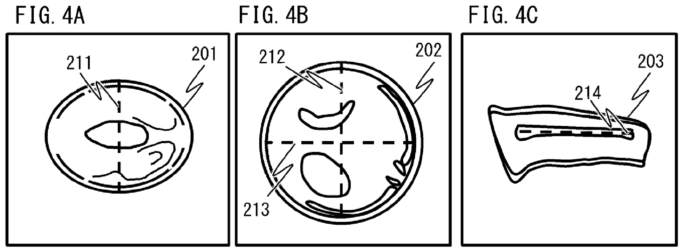

FIGS. 4A to 4C are diagrams schematically illustrating the above-described three types of standard cross sections estimated at the present step. FIG. 4A illustrates a standard cross section 201 of the head of the fetus estimated by the processing of this step. FIG. 4A indicates a measurement line 211 of a biparietal diameter of the fetus. By estimating the position and orientation of the standard cross section at this step, parameters of the standard cross section 201 of the fetal head, for which the measurement line 211 is defined, are obtained. FIG. 4B illustrates a standard cross section 202 of the abdomen of the fetus estimated by the processing of this step. FIG. 4B indicates a measurement line 212 of a transverse abdominal diameter and a measurement line 213 of an antero-posterior trunk diameter of the fetus. By estimation of the position and orientation of the standard cross section at the present step, parameters of the standard cross section 202 of the fetal abdomen, for which the measurement lines 212 and 213 are defined, are obtained. FIG. 4C is a diagram illustrating a standard cross section 203 of the fetal femur estimated at the present step. FIG. 4C indicates a measurement line 214 of the femur length of the fetus. By estimation of the position and orientation of the standard cross section at the present step, parameters of the standard cross section 203 of the fetal femur, for which the measurement line 214 is defined, are obtained.

(Step S140: Determine whether there is a standard cross section to be subjected to precise estimation) At step S140, on the basis of the position and orientation of each standard cross section estimated at step S130, the guidance information generation unit 43 determines, for each standard cross section, whether to perform precise estimation. Then, if there is at least one standard cross section for which precise estimation is determined to be performed (YES at S140), the guidance information generation unit 43 proceeds to step S150. If it is determined that precise estimation is not to be performed for any of the standard cross sections (NO at S140), the guidance information generation unit 43 proceeds to step S160 to calculate guidance information for assisting the user's probe operation.

The determination of whether to perform precise estimation on a standard cross section at the present step is made, for example, based on the movement amount from the center position of the three-dimensional volume to the estimated position of the center position of the standard cross section (that is, the distance between the two positions). For example, when the movement amount is large, specifically, when the movement amount is greater than the distance from the center position of the three-dimensional volume to the edge of the range of the three-dimensional volume, it is determined that guidance information for moving the probe needs to be generated and precise estimation is not performed. Here, the movement amount being greater than the distance from the center position of the three-dimensional volume to the edge of the range of the three-dimensional volume means that the center position of the standard cross section is not within the range of the three-dimensional volume unless the probe is moved. In contrast, when the movement amount is small, specifically, when the movement amount is less than or equal to the distance from the center position of the three-dimensional volume to the edge of the range of the three-dimensional volume, precise estimation is determined to be performed. Here, the movement amount being less than or equal to the distance from the center position of the three-dimensional volume to the edge of the range of the three-dimensional volume means that the standard cross section is located within the range of the three-dimensional volume.

In the above processing, whether guidance information generation processing is performed is switched depending on whether the center position of the standard cross section is within the range of the three-dimensional volume. However, even when the center position of the standard cross section is within the range of the three-dimensional volume, the guidance information generation unit 43 may determine not to perform precise estimation if the movement amount is greater than or equal to a predetermined distance. For example, the guidance information generation unit 43 may use 80% of the distance from the center position of the three-dimensional volume to the edge of the range as a threshold and determine whether to perform precise estimation based on whether the movement amount exceeds the threshold. In this case, even when the estimated position of the standard cross section calculated at step S130 is within the currently acquired three-dimensional volume image, it is determined that precise estimation is not to be performed if the estimated position is close to the edge of the image.

For example, after the generation of guidance information at step S160 described below, the precise estimation unit 44 may perform the precise estimation at step S150 when the user operates the probe on the basis of the guidance information and the standard cross section enters the imaging range. Thus, the user operates the probe such that the standard cross section of the observation target is imaged while checking the guidance information displayed on the display unit 36. Then, when the center position of the standard cross section is closer to the center of the image, the precise estimation unit 44 performs precise estimation. As a result, it is possible to expect improvement in the accuracy of estimation of standard cross section parameters by the precise estimation in a subsequent stage.

The image processing apparatus 10 according to the present embodiment may be configured such that, as the probe is moved closer to the estimated position of a predetermined cross section (for example, a standard cross section) by user probe operation based on guidance information (or mechanical control), an expectation value of estimation accuracy of the position and orientation of the predetermined cross section improves. Here, "an expectation value of estimation accuracy improves" does not simply mean that the estimated value is updated, but means that the uncertainty of the estimated value (such as error range, variation, confidence interval, variance of the estimated distribution, or uncertainty corresponding to the estimation score) is reduced, and the estimation result for the same predetermined cross section transitions to a state in which it is more stable and expected to be closer to the true position and orientation.

More specifically, each time the input image (an image of a three-dimensional volume or a two-dimensional sectional image) acquired by the acquisition unit 41 is updated during guidance in which the processing from step S120 to step S170 is repeated, the control unit 37 re-performs the estimation processing (step S130) by the estimation unit 42. Then, for the estimation result of the predetermined cross section obtained by the estimation unit 42, the control unit 37 utilizes the property that the reliability of the estimation result can increase as the cross section approaches a state relatively close to the current imaging range (for example, a state in which the cross section is included in the three-dimensional volume, the cross section is included in the vicinity of the boundary of the three-dimensional volume, or the cross section is close to a predetermined region within the plane of the two-dimensional sectional image), thereby improving the expectation value of estimation accuracy in a stepwise manner.

For example, the estimation unit 42 may generate, as estimation results, not only the estimated values of the position and orientation of a predetermined cross section (center position coordinates, rotation axis vector, rotation angle, and the like) but also an indicator representing the certainty of these estimated values (hereinafter referred to as "estimation confidence"). The estimation confidence may be an indicator based on, for example, an output score of the trained model, the variance of the output distribution, the variation of estimation results from multiple models (ensemble), uncertainty estimated by Monte Carlo dropout or the like, or features included in the input image (the visibility of anatomical structures defining the predetermined cross section).

Also, the estimation unit 42 may correct the estimation confidence on the basis of a distance indicator that indicates how close the predetermined cross section is to the current imaging range. For example, the distance indicator may be (i) the displacement amount from the center position of the three-dimensional volume to the center position of the estimated predetermined cross section, and/or (ii) the shortest distance to the estimated predetermined cross section from the three-dimensional volume boundary. Additionally or alternatively, the distance indicator may be (iii) the in-plane distance from the center of the two-dimensional sectional image to the center of the estimated predetermined cross section, and/or (iv) the probability that the estimated predetermined cross section is included in the imaging range, for example. The control unit 37 may calculate or correct the estimation confidence such that the estimation confidence increases as the distance indicator becomes smaller (that is, as the target position is approached).

Furthermore, the control unit 37 may sequentially integrate estimation results obtained during guidance so as to improve the expectation value of estimation accuracy of the target position. For example, the control unit 37 defines an estimation result (estimated value) obtained at time k as x_k, and a quantity representing the uncertainty of this estimation result (for example, covariance, variance, error radius, confidence interval width, or the like) as P_k. Each time a new input image is acquired during guidance, x_k and P_k are sequentially updated. The update processing may be achieved using a known technique such as a Kalman filter, an extended Kalman filter, a particle filter, an exponential moving average, Bayesian updating, or a weighted average based on estimation confidence. The control unit 37 can reduce variation in the estimation results and improve the expectation value of estimation accuracy of the target position in a stepwise manner by sequentially performing update processing so as to increase a weight applied to a newly obtained estimation result as the target position is approached (for example, as the distance indicator decreases).

Furthermore, in accordance with the progress status of guidance, the estimation unit 42 may improve the expectation value of estimation accuracy by changing the image region or resolution used for the estimation in a stepwise manner. For example, while the predetermined cross section is outside of the imaging range, the estimation unit 42 performs coarse estimation based on wide-area features. Then, at a stage when the predetermined cross section reaches the inside of the imaging range or the vicinity of the imaging range, the estimation unit 42 performs estimation (local estimation) by extracting, at a high resolution, a region in the vicinity of a candidate of the cross section, or performs estimation (iterative estimation) in which the estimation result is iteratively refined using a coarse estimation result as an initial value. Accordingly, as the target position is approached through guidance, the estimation error decreases and the expectation value of estimation accuracy improves.

Furthermore, even at a stage immediately before the conditions for executing the precise estimation unit 44 (step S150) are satisfied, it is conceivable that the estimation confidence of the estimation results generated by the estimation unit 42 exceeds a predetermined threshold. In such a case, the control unit 37 may introduce processing equivalent or similar to the estimation processing of the precise estimation unit 44 (for example, estimating using a high-accuracy model, increasing the number of iterations, narrowing the search range, or the like) in a stepwise manner to further improve the expectation value of estimation accuracy. That is, in the present embodiment, the estimation during guidance is not merely processing for updating the target position, but may be performed as processing for reducing the uncertainty of the estimation so that the expectation value of estimation accuracy increases as the target position is approached.

(Step S150: Perform precise estimation on standard cross section) At step S150, the precise estimation unit 44 estimates parameters representing the position and orientation of the standard cross section (standard cross section parameters) for the standard cross section for which precise estimation is determined to be performed at step S140 by the guidance information generation unit 43. When there are a plurality of standard cross sections for which precise estimation is determined to be performed at step S140, the precise estimation unit 44 sequentially performs the following processing at this step for each of the standard cross sections.

As described in step S120, the standard cross section parameters are represented by a total of seven numerical values, including three parameters of center position coordinates of the standard cross section, three parameters of rotation axis vector of the standard cross section, and one parameter of rotation angle of the standard cross section. At this step, the precise estimation unit 44 receives an image of the three-dimensional volume as input and estimates standard cross section parameters using a trained model that outputs standard cross section parameters. The estimation is performed based on a known standard cross section parameter estimation technique described in Non-Patent Literature 1.

After precise estimation by the precise estimation unit 44 is completed, the control unit 37 controls the display unit 36 via the display control unit 45 to notify the user that the precise estimation of the standard cross section has been completed. This notification processing is performed by the control unit 37 extracting the standard cross section based on the estimated standard cross section parameters and displaying the extracted standard cross section on the display unit 36. The control unit 37 may also display the extracted standard cross section together with a diagram relating to the standard cross section on the display unit 36. For example, the control unit 37 may display on the display unit 36 a schematic listing the standard cross sections to be acquired and display a check mark on the schematic representing a standard cross section for which precise estimation has been completed.

The position and orientation estimation processing of a standard cross section at this step may be achieved using any technique that inputs a three-dimensional image to a trained model and outputs a sequence of numerical values corresponding to the standard cross section. In the technique according to Non-Patent Literature 1, the expectation value of estimation accuracy of standard cross section parameters is increased in steps through iterative processing, but a technique may be employed in which the final estimation result is obtained in a single estimation. Furthermore, a trained model using any deep learning technique, such as Transformer, may be used instead of being limited to the convolutional neural network employed in the technique of Non-Patent Literature 1. Alternatively, a trained model using machine learning other than deep learning, such as an Active Shape Model, may be used.

Furthermore, after the notification of precise estimation and the estimation result is provided to the user at this step, the user may be allowed to select whether to confirm the estimation result. For example, when the user selects to confirm the estimation result of the precise estimation notified on the display unit 36, the precise estimation unit 44 stores the estimation result of the standard cross section in the RAM 33 and/or the storage unit 34. In contrast, when the user selects not to confirm the estimation result of the precise estimation notified on the display unit 36, the precise estimation unit 44 displays a user interface (UI) on the display unit 36 for the user to correct the estimation result, and receives correction instructions from the user. The precise estimation unit 44 then stores the estimation result of the standard cross section corrected according to the instruction received from the user in the RAM 33 and/or the storage unit 34.

Additionally, in the present step, an example has been described in which the precise estimation processing of the standard cross section is automatically performed when it is determined that "precise estimation is to be performed", but a processing flow that undergoes a confirmation process by the user may be used. In this case, for example, when precise estimation is determined to be performed, that is, when the input three-dimensional volume reaches the target site, a confirmation dialog such as "Acquire standard cross section?" is displayed. Then, the precise estimation of the standard cross section is performed only when the user presses the "Yes" button. This allows the user to intervene at an early stage when the determination processing at step S140 is incorrect, thereby advantageously reducing the working time.

(Step S160: Generate guidance information) At step S160, based on the information of the standard cross section estimated by the estimation unit 42, the guidance information generation unit 43 generates guidance information for guiding the probe so that a predetermined structure portion of the subject is included in the image. Specifically, the guidance information generation unit 43 generates guidance information to assist the user in moving the probe closer to the standard cross section for which guidance information is determined to be calculated at step S140. When there are a plurality of standard cross sections for which guidance information is determined to be calculated, the standard cross section having the smallest estimated movement amount from the three-dimensional volume center position to the standard cross section (that is, the standard cross section estimated to be closest to the three-dimensional volume center position) is selected as the target for generating guidance information.

Here, "guidance information" refers to information regarding the positional relationship between the current position of the probe and the position of the standard cross section. Specifically, the guidance information is reference information for the user to move the probe from the current position, such as "move the probe 10 cm toward the back". As such, the guidance information is information indicating the movement distance and the movement direction for moving the probe from the imaging position of the image to the position at which an image of a predetermined structure portion of the subject can be captured. Also, the generation of guidance information is performed by combining the estimated movement amount from the center position of the three-dimensional volume to the standard cross section and the parameter of "depth length" acquired by the image processing apparatus 10.

A specific example of processing for generating guidance information is now described. First, the guidance information generation unit 43 calculates relative coordinate values of the center position of the three-dimensional volume with respect to the tip position of the probe used by the user. These relative coordinate values can be calculated using the above-mentioned "depth length". This is because the depth length represents a range up to what depth from the body surface of the subject is imaged by imaging using the probe, and the center position of the three-dimensional volume is the center position of the imaged range.

Then, the guidance information generation unit 43 acquires, of the estimated values of the multiple standard cross sections calculated at step S130, the estimated values of a standard cross section for which guidance information is to be generated. Using the estimated values (that is, the relative position and orientation of the standard cross section from the center position of the three-dimensional volume), the guidance information generation unit 43 then calculates "the center position of the standard cross section from the tip position of the probe". The guidance information generation unit 43 then calculates "estimated values indicating in which direction and by what distance the probe should be moved on the body surface". These estimated values can be calculated by projecting the "center position of the standard cross section from the tip position of the probe" onto the body surface, assuming that the body surface in contact with the probe during imaging is locally a flat plane.

Furthermore, the guidance information generation unit 43 converts the guidance information generated by the above processing into the aforementioned text information and/or image information to display the guidance information on the display unit 36.

In this embodiment, a case is assumed in which the parameters of "depth length" acquired from the image processing apparatus 10 are used to generate guidance information, but the image processing apparatus 10 may generate guidance information by processing image information. For example, the guidance information generation unit 43 constructs a trained model that estimates the movement direction and the movement amount of the probe from the three-dimensional volume image and the estimated values of the standard cross section. Then, the guidance information generation unit 43 may input the three-dimensional volume image and the estimated values of the standard cross section calculated at step S130 to the trained model to generate information output from the trained model as guidance information. This provides an advantage in that the guidance information generation unit 43 can calculate the guidance information even when the formats of imaging parameters held by the image processing apparatus 10 and the probe are different, for example. Furthermore, the guidance information generation unit 43 may use a trained model trained to directly output guidance information (including movement direction and movement distance) when an image is input, and acquire guidance information output from the trained model. In this case, the guidance information generation unit 43 may generate guidance information without using the estimation result of information relating to a predetermined structure portion obtained by the estimation unit 42.

In the present embodiment, the guidance information generation unit 43 calculates "estimated values indicating in which direction and by what distance the probe should be moved on the body surface", but "estimated values indicating an amount by which the probe should be depressed" may be calculated. These estimated values represent the movement amount in the direction of probe depression presented to the user so that the three-dimensional volume can be acquired so as to include the standard cross section, when the standard cross section is positioned farther than the depth length in the direction of probe depression.

In the image processing apparatus 10 according to the present embodiment, among the plurality of standard cross sections, a standard cross section estimated to be closest to the center position of the three-dimensional volume is set as a target for the generation of guidance information. However, all of the plurality of standard cross sections may be set as targets for the generation of guidance information. In this case, the user may select a standard cross section for which guidance of the probe is desired with priority, and the image processing apparatus 10 may generate guidance information for the selected standard cross section. Alternatively, when the estimation unit 42 estimates the position and orientation of standard cross sections at step S130, not only parameters but also a score representing the confidence of the estimated position and orientation may be generated for each standard cross section. In this case, the guidance information generation unit 43 sets a standard cross section having the highest confidence as the target for generation of the guidance information.

(Step S170: Update display) At step S170, the display control unit 45 displays, on the display unit 36, information that visually presents to the user the guidance information generated at step S160. When the control unit 37 detects that the user has moved the probe on the basis of the guidance information displayed on the display unit 36, the control unit 37 returns the processing to step S120 and performs acquisition of the input image again.

The guidance information displayed on the display unit 36 is now described in detail. In the present embodiment, it is assumed that the guidance information such as "move the probe 10 cm toward the back" generated at step S160 is displayed as text information on the display unit 36. In this case, the user can determine the direction and amount of movement of the probe by referring to the text information of the guidance information displayed on the display unit 36. The guidance information display on the display unit 36 is maintained until the processing step of the control unit 37 reaches this step again and the display is updated.

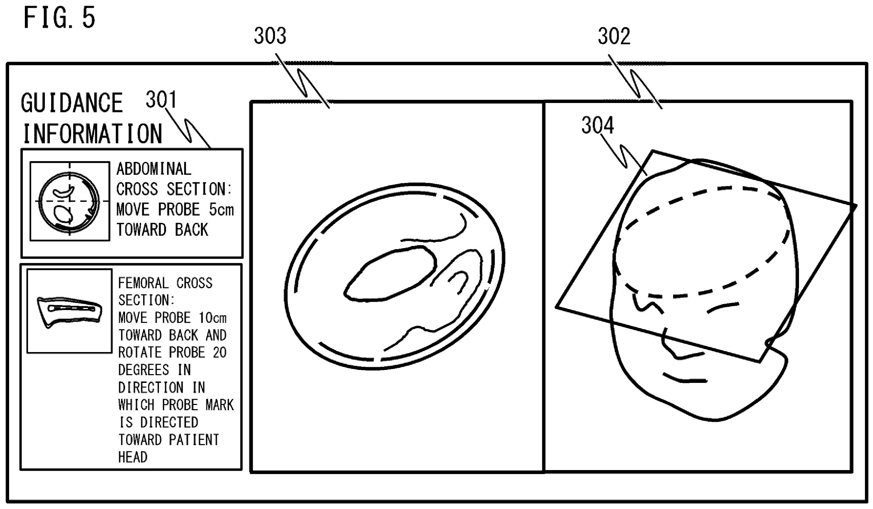

FIG. 5 illustrates an example of guidance information displayed on the display unit 36 at this step. Here, it is assumed that the user is viewing a standard cross section of the head of a fetus with the probe. The guidance information 301 generated at step S160 is displayed on the display unit 36. In the example of FIG. 5, the display unit 36 displays, as guidance information 301, information for guiding the user to move the probe so that a standard cross section of the abdomen of the fetus and a standard cross section of the femur of the fetus can be acquired. Also, as illustrated in FIG. 5, a volume rendering image 302 of a three-dimensional image of the fetus, which is an input image acquired at step S120, is displayed on the display unit 36. The display unit 36 also displays a standard cross section 303 of the head of the fetus currently being viewed by the user. Furthermore, a frame 304 indicating the position of the standard cross section 303 in the volume rendering image 302 is displayed on the display unit 36.

In this embodiment, guidance information combining various display forms other than text information may be generated and displayed on the display unit 36. For example, in addition to text information, the guidance information generation unit 43 may generate an icon in which the direction in which the probe moves is illustrated as guidance information, and the display control unit 45 may display the generated icon on the display unit 36. Alternatively, the guidance information generation unit 43 may generate audio information to assist the movement of the probe. In this case, the display control unit 45 causes a speaker or the like provided in the display unit 36 to emit a pulse sound having a long interval when the distance from the probe to the standard cross section is long, and to emit a pulse sound having a short interval when the distance from the probe to the standard cross section is short.

Furthermore, when all of the plurality of standard cross sections are set as targets for the generation of guidance information at step S160, the display control unit 45 may display guidance information in a different display form for each standard cross section, instead of displaying the guidance information for all of the standard cross sections in parallel on the display unit 36. For example, the display control unit 45 may display, in a larger size, the guidance information for the standard cross section for which the movement amount of the probe is the smallest, and display, in a smaller size, the guidance information for the other standard cross sections. Alternatively, the display control unit 45 may display, on the display unit 36, the guidance information other than that for the standard cross section having the smallest movement amount with increased transparency so as to be less noticeable. By controlling the display of the guidance information on the display unit 36, when the image processing apparatus 10 presents guidance information for a plurality of standard cross sections to the user, it is possible to achieve a more suitable display of guidance information that allows the user to easily understand the movement direction and movement amount of the probe.

(Step S180: Determine whether precise estimation is completed for all standard cross sections) At step S180, the control unit 37 determines whether precise estimation has been performed for all of the standard cross sections estimated at step S130 (in the present embodiment, all three types of standard cross sections of the head, the abdomen, and the femur of the fetus). When the control unit 37 determines that precise estimation is completed for all of the standard cross sections (YES at S180), the control unit 37 notifies the user of completion of the processing of this flowchart through the display unit 36 or the like, and ends the processing of this flowchart. When the control unit 37 determines that there is a standard cross section for which precise estimation has not been performed (NO at S180), the process returns to step S120 and acquisition of an input image is performed again.

The display control unit 45 may cause the display unit 36 to display, in addition to the guidance information 301 (for example, the movement direction and the movement amount) indicating the progress status of the guidance, information indicating the state of the expectation value of estimation accuracy of the predetermined cross section (target position) concurrent with the guidance progress. Also, the display control unit 45 may cause the display unit 36 to display information indicating whether precise estimation by the precise estimation unit 44 can be executed, or an execution preparation state. Here, information indicating whether precise estimation by the precise estimation unit 44 can be executed or the execution preparation state is also referred to as "precise estimation status information".

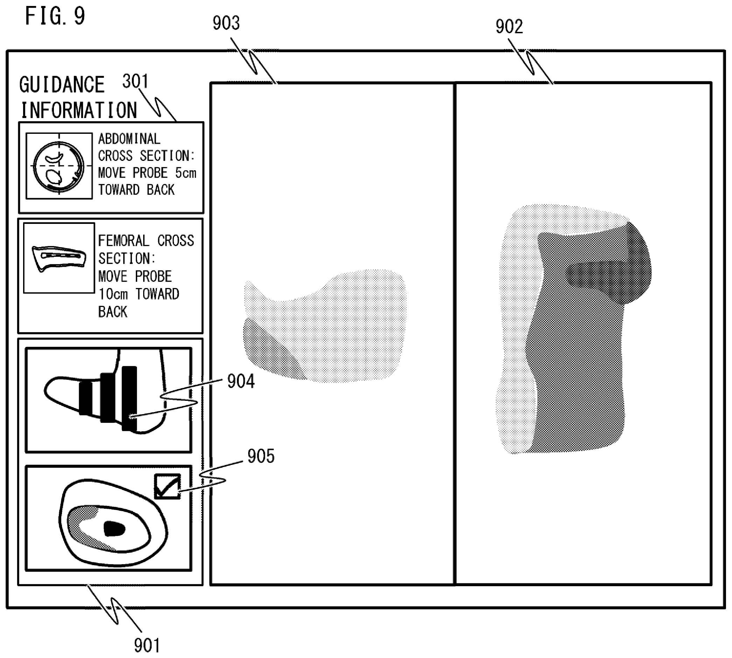

FIG. 9 is a diagram illustrating a display example of the precise estimation status information displayed on the display unit 36 by the image processing apparatus 10 according to the present embodiment. In the example illustrated in FIG. 9, the display unit 36 displays the precise estimation status information 901 on the same screen as the guidance information 301 (or on a related screen switchable to that screen). The precise estimation status information 901 may include, for example, estimation confidence of the target position, the magnitude of estimation error, a preparation state for transition to precise estimation, information on a cross section for which precise estimation is completed, and the like. Here, the target position may be a predetermined standard cross section, the magnitude of estimation error may be an error radius or a width of uncertainty, and the preparation state for transition to precise estimation may be "preparing for precise estimation", "precise estimation available", "precise estimation in progress", "precise estimation completed", or the like, for example.

FIG. 9 illustrates, as an example, a volume rendering image 902 of a femur and a standard cross section 903 in the volume rendering image 902, which are displayed on the display unit 36. The precise estimation status information 901 also displays a mark 904 as an example of an indicator indicating that precise estimation by the precise estimation unit 44 is in progress, as information relating to the progress status of precise estimation by the precise estimation unit 44. The mark 904 in the example of FIG. 9 consists of three vertical lines, the lengths of which increase from left to right. By the mark 904, the user can recognize that precise estimation is in progress for the cross section on which the mark 904 is superimposed. The precise estimation status information 901 also displays a check mark 905 as an example of an indicator indicating that precise estimation by the precise estimation unit 44 has been performed. By the mark 905, the user can recognize that precise estimation has been performed for the cross section on which the mark 905 is superimposed. Note that the marks 904 and 905 may be replaced with other appropriate indicators as long as the user can recognize that precise estimation is in progress and that precise estimation has been performed, respectively.

For example, the estimation confidence may be a score associated with the estimation result output by the estimation unit 42, or may be calculated by the control unit 37 on the basis of the output of the estimation unit 42 and a distance indicator (for example, the displacement amount from the three-dimensional volume center to the estimation center). The magnitude of an estimation error may be a variance of the output distribution of the estimation unit 42, ensemble variation, time-series fluctuation of estimated values, or an error range converted from the estimation confidence.

The display control unit 45 may change the display format of the precise estimation status information 901 according to the estimation confidence or the magnitude of estimation error. For example, when the estimation confidence is low, the display control unit 45 displays the precise estimation status information 901 with a display such as "low" or "guidance required" (or with a first color or a first transparency). Also, for example, when the estimation confidence is high, the display control unit 45 displays the precise estimation status information 901 with a display such as "high" or "precise estimation available" (or with a second color or a second transparency). Alternatively, the display control unit 45 may display the estimation confidence as a numerical value (for example, 0 to 1 or 0 to 100). Alternatively, the display control unit 45 may display the magnitude of estimation error as an error radius (for example, in units of mm or cm).

Furthermore, the display control unit 45 may perform a display that visualizes, along a time series, an increase in the estimation confidence or a reduction in the estimation error so that the user can understand that the expectation value of estimation accuracy is improving with the progress of the guidance. For example, the display control unit 45 may extend a gauge display (a progress bar) in accordance with an increase in the estimation confidence, or may display a graphic of the error range (for example, a circle, an ellipse, a frame, or a band-shaped region) in a smaller size in accordance with a reduction in the estimation error. As a result, the user can visually understand that the expectation value of estimation accuracy is increasing as the probe approaches the target position by operating the probe according to the guidance information 301.

Furthermore, the display control unit 45 may perform a display that assists the user operation concerning the execution timing of the precise estimation unit 44. For example, when the estimation confidence is greater than or equal to a predetermined threshold and it is determined that a predetermined cross section is likely to be within the imaging range, the display control unit 45 may display "precise estimation available" or a similar display, and may prompt the user to stop or slow the movement of the probe. As a result, it is easier to ensure image quality (for example, reduced blur) of the input image supplied to the precise estimation unit 44, and improvement in accuracy of the precise estimation can be expected.

When there are a plurality of guidance targets (for example, abdominal cross section and femoral cross section), the precise estimation status information 901 illustrated in FIG. 9 may be displayed separately for each guidance target, or may be displayed only for the guidance target selected by the user. Alternatively, the display control unit 45 may perform a display in which the estimation confidence and the estimation error for each guidance target are presented in parallel so that they can be compared. Furthermore, when there are a plurality of guidance targets, a configuration may be adopted in which the user specifies an order of guidance.

Referring to the flowchart of FIG. 6, the acquisition processing of the trained model at step S110 is now described in detail.

(Step S1110: Acquire training image) At step S1110, the estimation unit 42 acquires training images to be used for training of a trained model from a database (not illustrated). In the same manner as the image of a three-dimensional volume at step S120, the images acquired at this step are images of parts of subjects. However, each image is obtained by performing imaging multiple times for one subject while changing the probe position. Accordingly, an image of the entire region of the subject can be obtained by stitching together the captured images. The training images acquired at this step are images obtained by imaging a large number of subjects using the above imaging method.

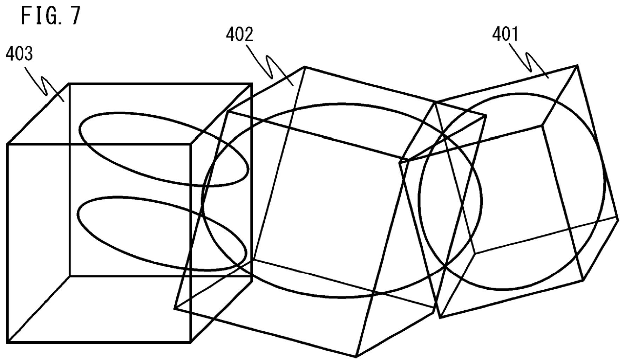

FIG. 7 illustrates an example of training images acquired at this step. Through processing of this step, a three-dimensional image 401 of a partial region obtained by imaging a head region of a fetus, a three-dimensional image 402 of a partial region obtained by imaging an abdominal region of the fetus, and a three-dimensional image 403 of a partial region obtained by imaging a leg region of the fetus are acquired. As schematically illustrated in FIG. 7, the imaging ranges of the three-dimensional images 401 and 402 partially overlap each other, and the imaging ranges of the three-dimensional images 402 and 403 partially overlap each other. Accordingly, an image of the entire region of the fetus can be obtained by stitching together the three three-dimensional images 401, 402, and 403.

(Step S1120: Perform stitching of training images) At step S1120, the estimation unit 42 performs stitching (joining) of the training images for each subject. As described in step S1110, the training images are images obtained by performing imaging multiple times for one subject. Accordingly, at this step, the estimation unit 42 obtains an image of the entire region of the subject by combining a plurality of images for each subject.

For the stitching at this step, various known techniques may be used. For example, Scale-Invariant Feature Transform (SIFT) or Speeded-Up Robust Features (SURF) may be used. In these techniques, image feature points are extracted from the images, and positions having matching features are matched. Alternatively, a trained model based on deep learning that has been trained to output an image that is a stitching result from a plurality of input images may be used.

In this manner, through the processing of this step, an image including the entire region of the subject is generated. The estimation unit 42 performs the above processing for each of the images of a plurality of subjects acquired at step S1110, thereby generating, for each subject, an image including the entire region of the subject.

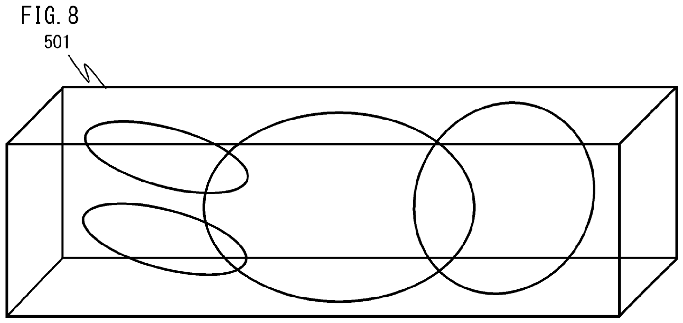

FIG. 8 schematically illustrates an example of an image including the entire region of a fetus that is generated by performing the stitching of this step on the training images illustrated in FIG. 7. As illustrated in FIG. 8, through the processing of this step, the three-dimensional images 401, 402, and 403 illustrated in FIG. 7 are combined, and an image 501 of the entire region of the fetus is generated.

At step S1110, for example, a magnetic or optical tracking marker may be attached to the probe so that the estimation unit 42 may acquire information indicating the relative relationship of the position and orientation of the probe when each image is captured. In this case, the control unit 37 acquires information about the tracking marker and transmits, to the probe, information for mechanically controlling the operation of the probe on the basis of the acquired tracking marker information. As a result, the control unit 37 acquires a series of three-dimensional volume images of the subject captured by the probe. In this case, the estimation unit 42 performs stitching of the training images by combining and arranging the respective three-dimensional volume images in a three-dimensional space on the basis of the position and orientation of the probe determined from the acquired information.

(Step S1130: Acquire ground-truth information) At step S1130, the estimation unit 42 acquires ground-truth information set by the user for the images generated at step S1120.

Here, the ground-truth information refers to standard cross section parameters for each of the head, abdomen, and femur of a fetus. At this step, the user sets the standard cross section parameters for each image, and the setting information is acquired. Specifically, the display control unit 45 causes the display unit 36 to display a multiplanar reconstruction (MPR) sectional image of the three-dimensional volume stitched at step S1120. The user then interactively sets the standard cross sections by changing the position and/or orientation of the MPR cross section displayed on the display unit 36 through operations of the mouse or keyboard. The estimation unit 42 then acquires the standard cross sections of the head, abdomen, and femur set by the user as ground-truth information for one case.

The setting and acquisition of training data to be used for training of a trained model may be performed in the image processing apparatus 10 or may be implemented as a function of an ultrasonic diagnostic apparatus external to the image processing apparatus 10. Alternatively, the setting and acquisition of training data may be achieved by a computer different from the above apparatuses.

(Step S1140: Generate training data) At step S1140, the estimation unit 42 generates training data (labeled data) for constructing a trained model by using the training images and the ground-truth information acquired through the processing of steps S1110 to S1130.

A specific example of a method for generating the training data at this step is now described. First, the estimation unit 42 sets a random position, orientation, and size for an arbitrary training image, and extracts a partial region image from the training image on the basis of the settings. The training image for extraction is an image including a range that is larger than the range captured by a single imaging operation using the probe. Then, the estimation unit 42 calculates, from the origin of the extracted partial region image, the relative position and orientation to the ground-truth data of each standard cross section in the original training image (relative values of the ground-truth data). Here, each standard cross section may be located outside of the range of the partial region image. A set of the generated partial region image and the relative values of the ground-truth data is treated as one piece of training data. The estimation unit 42 generates training data of the present embodiment by performing this training data generation processing multiple times (for example, 500 times) for one training image and further performing the generation processing for all training images.

(Step S1150: Construct trained model) At step S1150, the estimation unit 42 constructs a trained model using the training data generated at step S1140. Examples of the trained model constructed at this step include a trained model based on known deep learning such as VGG16 and ResNet. As long as the standard cross section parameters can be output by inputting a three-dimensional volume, a trained model based on machine learning that does not use deep learning, such as principal component analysis, may also be employed.

Thus, in specifying the standard cross sections of the subject, the above processing allows for the estimation of the position and orientation of a standard cross section that is located outside of the three-dimensional volume, even when the range of the three-dimensional volume covers only a partial region of the subject. Accordingly, guidance information can be output to enable the user to operate the probe so that the standard cross section is included within the three-dimensional volume, thereby reducing the user's burden during diagnosis.

In the present embodiment, it is assumed that the probe operation is guided by the interactive operation with the user to specify the position and orientation of the standard cross section, but the present embodiment is also applicable to a case in which the probe is automatically guided without involving the user operation. In this case, the probe is mounted on a robotic arm or jig and is driven under mechanical control. When guidance information is generated by the processing of the present embodiment, the probe is driven by mechanical control based on the guidance information. In this case, there is an advantage that the generated guidance information is accurately reflected in the motion of the probe because the operation by the user is not involved. In this case, the display processing of guidance information performed by the display control unit 45 at step S170 may be omitted.

Although the present embodiment has been described by exemplifying a case in which standard cross section parameters are estimated, any information other than standard cross section parameters may be the target for estimation as long as the information can be specified from an image of the subject. For example, the estimation target may be the movement amount of the probe required for a predetermined structure portion, such as a specific chamber of the fetal heart, to be included within the three-dimensional volume range.

Furthermore, in the present embodiment, the image processing apparatus 10 may perform, in the background, processing such as the generation of guidance information and the estimation of standard cross sections described above, instead of presenting the user with the movement direction and the movement amount of the probe. In this case, the control unit 37 continuously monitors images acquired while the user freely moves the probe and estimates the standard cross section parameters. For example, when a standard cross section for which precise estimation is determined to be possible appears within the three-dimensional volume while the user moves the probe, the control unit 37 internally performs precise estimation. The display control unit 45 then draws a check mark on the schematic representing the standard cross section displayed on the display unit 36. This allows the user to check the standard cross section corresponding to the schematic at any timing via the display unit 36.

Furthermore, when the above processing is performed in the background, the image processing apparatus 10 may receive user instructions for some of the processing, instead of performing the entire processing without notifying the user. For example, when a standard cross section for which precise estimation is determined to be possible appears within the three-dimensional volume while the user moves the probe, the display control unit 45 may display guidance information on the display unit 36 to prompt the user to reduce the probe's moving speed. As a result, the user can move the probe more slowly based on the information displayed on the display unit 36, thereby acquiring a three-dimensional volume image of higher quality. Consequently, the image processing apparatus 10 can achieve more accurate estimation of the standard cross sections. Thus, the image processing apparatus 10 enables both free operation of the probe by the user and high-accuracy estimation of the standard cross sections.

In the present embodiment, the control unit 37 may omit step S150 and determine the standard cross sections on the basis of manual operation by the user. Also, in the present embodiment, different trained models are used for the trained model used at step S130 and the trained model used at step S150. However, the same trained model may be used in the processing of both steps. Accordingly, the control unit 37 may perform precise estimation at step S150 using the trained model used at step S130.

At step S140 (determining whether there is a standard cross section for precise estimation) in the present embodiment, when the movement amount (displacement amount) from the center position of the three-dimensional volume image to the estimated position of the center position of the standard cross section is less than or equal to the predetermined value, precise estimation is determined to be performed. However, even when the displacement amount is less than or equal to the predetermined value, the control unit 37 may determine to generate guidance information instead of performing precise estimation, until the position of the probe approaches the standard cross section. In this case, the guidance information generation unit 43 generates, as guidance information, not only information related to the movement of the probe but also information related to imaging parameters such as the depth length or scale. Accordingly, the guidance information generation unit 43 generates, as guidance information, not only the movement amount of the probe but also the orientation of the probe (for example, the orientation when the user holds the probe and places it against the body of the subject).

As such, when the displacement amount of the estimated position and orientation of the predetermined cross section relative to the position of the three-dimensional volume is greater than a predetermined value, the guidance information generation unit 43 generates guidance information to bring the predetermined cross section within the three-dimensional volume. When the displacement amount is less than or equal to the predetermined value, the guidance information generation unit 43 generates guidance information such that the position and orientation of the predetermined cross section satisfy the predetermined conditions for imaging of the predetermined cross section by the probe in the three-dimensional volume.

Specifically, the guidance information generation unit 43 refers to the result of the precise estimation of the standard cross section at step S150. When the estimated position of the standard cross section is farther from the center position of the current three-dimensional volume image than a predetermined distance, the guidance information generation unit 43 generates guidance information for adjusting the imaging parameters. This allows the image processing apparatus 10 to present the user with guidance information for guiding the probe to a position closer to the standard cross section. Thus, improvement in accuracy of the precise estimation can be expected.

### First Modification

A first modification of the present embodiment is now described. In the following description, configurations that are the same as those in the first embodiment will be given the same reference numerals, and detailed description thereof will be omitted.

In the image processing apparatus 10 according to the first embodiment, the position and orientation of the standard cross section are targets for the generation of guidance information and precise estimation. In contrast, in the image processing apparatus 10 according to the present modification, only the position of the standard cross section is the target for standard cross section estimation, generation of guidance information, and precise estimation. In this case, in the image processing apparatus 10, only the position of the standard cross section is acquired in the standard cross section estimation at step S130 and the precise estimation of the standard cross section at step S150. Subsequently, the orientation of the standard cross section is adjusted according to manual operation performed by the user on the image processing apparatus 10, and the standard cross section parameters are determined.

Also, in this modification, the target of precise estimation may be the position of a specific site, such as the thalamus or lateral ventricle in the fetal head, rather than the standard cross section, and the target for guidance information generation and precise estimation may be the position of the standard cross section in the image processing apparatus 10. Accordingly, in this modification, the estimation unit 42 estimates, based on the input image, information indicating the position of a predetermined structure portion (site) of the subject located outside of the imaging range of the image. In this case, once the position of the target site is determined through precise estimation, the precise estimation processing for that structure portion is completed. Thus, the orientation adjustment processing in the first embodiment is unnecessary, and it can be expected that the processing load of the image processing apparatus 10 is reduced.

### Second Modification

A second modification of the present embodiment is now described. In the following description, configurations that are the same as those in the first embodiment will be given the same reference numerals, and detailed description thereof will be omitted.

In the image processing apparatus 10 according to the first embodiment, the various types of processing described above are performed using a three-dimensional ultrasound image of a fetus. In contrast, in the image processing apparatus 10 according to the present modification, the various types of processing in the first embodiment are performed using three-dimensional medical images of a subject other than a fetus.

Specific examples of the present modification include a situation where a cross section for observing the aortic valve or the mitral valve is estimated in a three-dimensional cardiac region image (3D transesophageal echocardiographic image) captured using a transesophageal ultrasound probe. Since a transesophageal ultrasound probe moves inside of the esophagus, its degree of freedom of movement is less than that of an abdominal probe. However, the imaging range can be changed by bending the transesophageal ultrasound probe and by changing the rotation angle of the transducer at the probe tip. Accordingly, when the image processing apparatus 10 according to the present modification performs the various types of processing of the first embodiment using a 3D transesophageal echocardiographic image, information for adjusting translation and bending of the probe and the rotation angle of the transducer is generated as guidance information.

As such, according to the image processing apparatus 10 of the present modification, it is possible to perform the same processing as in the first embodiment using an image of a subject other than a fetus captured with a three-dimensional abdominal probe.

### Second Embodiment

A second embodiment is now described. In the following description, configurations that are the same as those in the first embodiment will be given the same reference numerals, and detailed description thereof will be omitted.

In the first embodiment, a case is assumed in which the input image is a three-dimensional volume image and the position and orientation of a plurality of two-dimensional sectional images within the three-dimensional volume image are acquired. In contrast, in the image processing apparatus 10 according to the present embodiment, a case is assumed in which the input image is a two-dimensional image, that is, a subject is imaged using a two-dimensional ultrasound probe, and precise estimation is performed on the position and orientation of a plurality of two-dimensional sectional images of the subject.

In the present embodiment, a case is assumed in which the imaging target is the heart, and various cross sections used for diagnosing various regions of the heart, such as four-chamber view, two-chamber view, three-chamber view, short-axis view, and parasternal long-axis view, are acquired. When the heart is the imaging target, the manner of expansion and contraction of each chamber and the movement of the valves varies depending on the time point in the cardiac cycle at which the image is captured. In the present embodiment, as one example, imaging is performed at the end-diastolic phase of the left ventricle. In the following description, it is assumed that an input image to be acquired is an image captured with timing aligned with the end-diastolic phase by electrocardiogram synchronization.

The configuration of the image processing apparatus 10 of the present embodiment is similar to that of the image processing apparatus 10 according to the first embodiment illustrated in FIG. 1. Also, the image processing apparatus 10 of the present embodiment performs processing according to the flowcharts illustrated in FIGS. 2 and 6, similarly to the first embodiment. However, in the image processing apparatus 10 according to the present embodiment, the processing content of each processing unit and each step differs from that of the first embodiment. As such, the functions of the processing units and the processing contents of the steps in the present embodiment are described, focusing on differences from the functions of the processing units and the processing contents of the steps in the first embodiment.

The acquisition unit 41 acquires, as an input image, a two-dimensional sectional image obtained by imaging a part of the cardiac region. Processing performed by the acquisition unit 41 is the same as that of the first embodiment except that an image to be acquired is a two-dimensional sectional image.

Using a two-dimensional sectional image as an input image, the estimation unit 42 estimates the position and orientation of the plurality of standard cross sections including those located outside of the cross section.

As in the first embodiment, the guidance information generation unit 43 determines whether to generate guidance information for assisting the user's probe operation for each of the standard cross sections. Then, the guidance information generation unit 43 generates and outputs guidance information for the standard cross section for which guidance information is determined to be generated.

As in the first embodiment, the precise estimation unit 44 estimates parameters representing the position and orientation of the standard cross section for which the guidance information generation unit 43 has determined not to generate guidance information.

As in the first embodiment, the display control unit 45 performs control for displaying on the display unit 36 the guidance information generated by the guidance information generation unit 43, the results of the estimation performed by the precise estimation unit 44, and images used for observing the subject when the user moves the probe on the basis of the guidance information.

Referring to the flowchart of FIG. 2, an example of processing performed by the image processing apparatus 10 in the present embodiment is now described. The processing of step S170 and step S180 is the same as that in the first embodiment.

(Step S110: Acquire trained model) At step S110, the estimation unit 42 acquires a trained model that estimates, from a two-dimensional sectional image, the position and orientation of a plurality of standard cross sections including a standard cross section located outside of the two-dimensional sectional image. The estimation unit 42 then stores the acquired trained model in the RAM 33.

The trained model used in the present embodiment is a model that estimates the positions and orientations of five types of standard cross sections in the cardiac region, namely a four-chamber view, a two-chamber view, a three-chamber view, a short-axis view, and a parasternal long-axis view, which are standard cross sections in the cardiac region. The two-dimensional sectional image currently being captured is input to the trained model, and a relative position and a relative angle from the input two-dimensional sectional image are estimated for each standard cross section.

Compared with the first embodiment, the present embodiment differs in that a two-dimensional sectional image, rather than a three-dimensional volume image, is input to the trained model. On the other hand, the relative position and orientation of each standard cross section output by the trained model in the present embodiment are, as in the first embodiment, seven parameters per standard cross section, that is, parameters representing the position and orientation in a three-dimensional space. Accordingly, in the present embodiment, to generate guidance information such as "translate 10 cm in the depth direction from the current sectional image", the displacement amount of the position and orientation in the forward direction or the depth direction of the two-dimensional sectional image is also obtained as an output of the trained model.

A specific method for constructing a trained model acquired at this step will be described below using the flowchart of steps S1110 to S1150 illustrated in FIG. 6.

(Step S120: Acquire input image) At step S120, the acquisition unit 41 acquires from the ultrasonic diagnostic apparatus 22 a two-dimensional sectional image capturing a partial region of the heart of a subject. The acquisition unit 41 then stores the acquired two-dimensional sectional image in the RAM 33.

(Step S130: Estimate standard cross section) At step S130, the estimation unit 42 inputs the input two-dimensional sectional image acquired at step S120 to the trained model acquired at step S110, and estimates the position and orientation of a plurality of standard cross sections. The estimation unit 42 stores parameters of each standard cross section acquired by the estimation in the RAM 33.

The type and representation format of the standard cross sections are as described in step S110. As described above, although the input image is a two-dimensional sectional image, the parameters of the estimated standard cross section represent the position and orientation in three-dimensional space. Also, as in the first embodiment, when this step is performed again after step S150, which will be described below, the control unit 37 assigns, to a standard cross section for which precise estimation has already been performed at step S150, an identifier indicating that estimation of the standard cross section has been completed, for example, an identifier "finalized". Then, the control unit 37 excludes the standard cross section with the "finalized" identifier from the targets for the estimation processing at the present step and the processing at subsequent steps.

(Step S140: Determine whether there is a standard cross section to be subjected to precise estimation) At step S140, on the basis of the position and orientation of each standard cross section estimated at step S130, the guidance information generation unit 43 determines, for each standard cross section, whether to perform precise estimation, as in the first embodiment. Then, if there is at least one standard cross section for which precise estimation is determined to be performed (YES at S140), the guidance information generation unit 43 proceeds to step S150. If it is determined that precise estimation is not to be performed for any of the standard cross sections (NO at S140), the guidance information generation unit 43 proceeds to step S160 to calculate guidance information for assisting the user's probe operation.

The difference between the determination processing at this step and that of the first embodiment is now described. The present embodiment differs from the first embodiment in that the determination is performed based on the movement amount from the center position of the two-dimensional sectional image to the estimated position, but the determination result changes depending on the movement "direction". For example, when a standard cross section is estimated to be located on the "front" or "back" side of the two-dimensional sectional image, the standard cross section cannot be brought within the two-dimensional sectional image unless the probe is moved, even if the movement amount is small. Also, even if the position of the standard cross section can be brought within the two-dimensional sectional image only by in-plane movement of the two-dimensional sectional image, it is necessary to move the probe when the movement amount of the image is large.

Accordingly, the guidance information generation unit 43 performs determination processing at this step considering not only the movement "amount" but also the movement vector, that is, the "direction" of movement. For example, when the vector of movement of a certain standard cross section to the estimated position represents a forward direction or a depth direction of the two-dimensional sectional image, that is, a movement direction "other than within the plane", the guidance information generation unit 43 determines that "precise estimation is not to be performed" for that standard cross section regardless of the movement amount. Also, even when the vector represents movement within the plane of the two-dimensional sectional image, the movement amount may be greater than or equal to half of the width of the defined range of the image, for example. In this case, the structure portions that should appear in the standard cross section (for example, in the case of a four-chamber view, the four cardiac chambers of the left ventricle, left atrium, right ventricle, and right atrium) are unlikely to be within the two-dimensional sectional image. Accordingly, the guidance information generation unit 43 determines that "precise estimation is not to be performed" for this standard cross section also in such a case.

(Step S150: Perform precise estimation on standard cross section) At step S150, as in the first embodiment, the precise estimation unit 44 estimates parameters representing the position and orientation of the standard cross section (standard cross section parameters) for the standard cross section for which precise estimation is determined to be performed by the guidance information generation unit 43.

Unlike the first embodiment, the standard cross section parameters estimated at this step represent the position and orientation within a two-dimensional sectional image, rather than the position and orientation in three-dimensional space. Specifically, the standard cross section parameters are given by a total of three parameters, namely, two parameters representing the center position coordinates of the standard cross section, and one parameter representing the rotation angle of the standard cross section.

At this step, a trained model is used that receives a two-dimensional sectional image as input and outputs three standard cross section parameters. The estimation processing of the position and orientation of the standard cross section at this step is performed, similarly to the first embodiment, based on the known technique described in Non-Patent Literature 1. In this case, except that the number of standard cross section parameters to be estimated is three instead of seven, the processing is the same as that of the first embodiment.

(Step S160: Generate guidance information) At step S160, the guidance information generation unit 43 generates guidance information to assist the user in moving the probe closer to the standard cross section for which guidance information is determined to be calculated at step S140, as in the first embodiment. Except for the difference that the images captured by the probe are two-dimensional images instead of three-dimensional images, the processing for generating the guidance information is the same as that of the first embodiment.

Referring to the flowchart of FIG. 6, the acquisition processing of the trained model at step S110 in the present embodiment is now described in detail.

(Step S1110: Acquire training image) At step S1110, the estimation unit 42 acquires training images to be used for training of a trained model from a database (not illustrated). In the present embodiment, although the input image acquired at step S120 is a two-dimensional sectional image, the training images acquired at this step are images of a three-dimensional volume, similarly to the first embodiment. As described in step S130, to calculate guidance information in the present embodiment, it is necessary to estimate "the position and orientation of the two-dimensional sectional image in a three-dimensional space". To this end, the training images acquired at this step need to be images of a three-dimensional space.

Also, at this step, the estimation unit 42 performs image processing on the acquired training images to correct "the difference in the luminance value distribution between the three-dimensional volume image and the two-dimensional sectional image". Due to differences in the probe or the image reconstruction function, simply extracting a two-dimensional cross section from a three-dimensional volume image results in a luminance value distribution in the extracted sectional image that differs from the luminance value distribution in the two-dimensional sectional image captured by the probe. For this reason, when an image of a two-dimensional cross section that is simply extracted from a three-dimensional volume image is used as a training image for training of a trained model, the estimation accuracy at step S130 may be reduced. As such, in this embodiment, the estimation unit 42 performs, on an image of a two-dimensional cross section extracted from a three-dimensional volume image, processing to bring the luminance value distribution closer to the average luminance value distribution of two-dimensional sectional images by changing the window level or window depth. The estimation unit 42 may convert the luminance values using a known image generation model such as a Generative Adversarial Network (GAN).

When the imaging target is the subject's heart as in this embodiment, it is expected that training of the trained model can be performed more accurately by causing the cardiac cycle to be reflected in the processing of acquiring training images. That is, depending on the imaging timing, the time point in the cardiac cycle at which the image is captured varies. Therefore, in this embodiment, the estimation unit 42 uses the electrocardiogram data of the subject acquired from outside of the image processing apparatus 10 to acquire training images by capturing images at the timing of the phase (end-diastolic phase) targeted by the image processing apparatus 10.

The training image may be a two-dimensional sectional image captured by a two-dimensional ultrasonic probe. In this case, a series of time-series images obtained by imaging a single subject while gradually moving the probe are used as training images. A magnetic or optical tracking marker is attached to the probe used for imaging the training images, and the estimation unit 42 acquires the captured images in association with information on the position and orientation of the probe when the images are captured.

(Step S1120: Perform stitching of training images) At step S1120, the estimation unit 42 performs stitching (joining) of the training images. In this embodiment, the training image acquired at step S1110 is a three-dimensional volume image, as in the first embodiment. The processing of this step is therefore similar to the processing of step S1120 in the first embodiment.

Depending on the imaging conditions for training images at step S1110, the processing at this step may not be necessary. That is, when a three-dimensional volume image covering the entire cardiac region can be acquired in a single imaging operation at step S1110, the estimation unit 42 omits the stitching (joining) processing at this step.

Furthermore, when the training images acquired at step S1110 are two-dimensional sectional images, the estimation unit 42 places each two-dimensional sectional image in a virtual three-dimensional space on the basis of the measurement results output from the tracking marker attached to the probe. Then, the estimation unit 42 performs this processing for each subject, thereby obtaining an image that is the stitching result.

(Step S1130: Acquire ground-truth information) At step S1130, the estimation unit 42 acquires ground-truth information set by the user for the images generated at step S1120, as in the first embodiment. Here, the ground-truth information refers to five types of standard cross section parameters in the cardiac region, namely a four-chamber view, a two-chamber view, a three-chamber view, a short-axis view, and a parasternal long-axis view of the heart of the subject. The processing of acquiring the ground-truth information at this step is the same as the process at step S1130 in the first embodiment.

(Step S1140: Generate training data) At step S1140, the estimation unit 42 generates training data (labeled data) for constructing a trained model by using the training images and the ground-truth information acquired through the processing of steps S1110 to S1130.

As compared with the processing at step S1140 of the first embodiment, the processing of this step differs in that the partial region image extracted from the stitching result image acquired at step S1120 is a two-dimensional sectional image rather than an image of a three-dimensional volume.

(Step S1150: Construct trained model) At step S1150, the estimation unit 42 constructs a trained model using the training data generated at step S1140. The processing of this step is similar to the processing of step S1150 in the first embodiment.

According to the image processing apparatus 10 according to the present embodiment, even when the input image is not an image of a three-dimensional volume but a two-dimensional sectional image, it is possible to estimate the position and orientation of a standard cross section located outside of the defined range of the sectional image. As a result, similar to the first embodiment, guidance information that assists the user in operating the probe is presented to the user, and it is expected that the burden on the user during diagnosis is reduced.

### Third Modification

A third modification, which is a modification of the second embodiment, is now described. In the following description, configurations that are the same as those in the second embodiment will be given the same reference numerals, and detailed description thereof will be omitted.

In the second embodiment, a case is assumed in which a two-dimensional ultrasonic image, which is a medical image obtained by imaging the heart of a subject, is the target for precise estimation. In contrast, in this modification, two-dimensional images other than medical images are the targets for precise estimation.

A specific example of this modification includes a case in which the image processing apparatus 10 acquires a captured image from a robot including a camera while the robot moves in a building, performs processing similar to the above using the acquired image, and performs precise estimation for navigation of the robot. In this case, it is assumed that an input image to the trained model is a two-dimensional image captured by the camera of the robot, and that an image of a three-dimensional volume of the building in which the robot moves is acquired in advance.

The control unit 37 of the image processing apparatus 10 acquires a captured image from the robot as an input image at step S120, and performs the processing of steps S130 to S180 using the acquired input image. Thus, even when the target position of the movement destination of the robot is located outside of the range of the input image, the image processing apparatus 10 can generate estimated values of the movement amount and movement direction as guidance information. Consequently, by transmitting the generated guidance information to the robot, the image processing apparatus 10 can perform navigation of the robot's movement.

Note that the above-described various types of control may be processing that is carried out by one piece of hardware (e.g., processor or circuit), or otherwise. Processing may be shared among a plurality of pieces of hardware (e.g., a plurality of processors, a plurality of circuits, or a combination of one or more processors and one or more circuits), thereby carrying out the control of the entire device.

Also, the above processor is a processor in the broad sense and includes general-purpose processors and dedicated processors. Examples of general-purpose processors include a central processing unit (CPU), a micro processing unit (MPU), a digital signal processor (DSP), and so forth. Examples of dedicated processors include a graphics processing unit (GPU), an application-specific integrated circuit (ASIC), a programmable logic device (PLD), and so forth. Examples of PLDs include a field-programmable gate array (FPGA), a complex programmable logic device (CPLD), and so forth.

The embodiment described above (including variation examples) is merely an example. Any configurations obtained by suitably modifying or changing some configurations of the embodiment within the scope of the subject matter of the present disclosure are also included in the present disclosure. The present disclosure also includes other configurations obtained by suitably combining various features of the embodiment.

### Other Embodiments

Embodiment(s) of the present disclosure can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)^{™}), a flash memory device, a memory card, and the like.

Various embodiments have been described in detail above but it will be understood that the present disclosure is not limited to these embodiments and encompasses all modifications, variants, alternatives and equivalents falling within the scope of the appended claims.

## Claims

1. An image processing apparatus comprising:
acquisition means configured to acquire an image that represents a part of a subject and is captured using a probe; and
generation means configured to generate, on the basis of the image, guidance information for guiding the probe such that a predetermined structure portion of the subject located outside of an imaging range of the image is included within the imaging range.

2. The image processing apparatus according to claim 1, further comprising:
estimation means configured to estimate information relating to a position of the predetermined structure portion on the basis of the image, wherein
the generation means generates the guidance information on the basis of the information estimated by the estimation means.

3. The image processing apparatus according to claim 2, wherein
the estimation means estimates a position and an orientation of the predetermined structure portion as the information, and
the generation means generates the guidance information on the basis of the position and the orientation estimated by the estimation means.

4. The image processing apparatus according to claim 2, wherein
the estimation means estimates, as the information, a position at which a standard cross section of the predetermined structure portion is imaged, and
the generation means generates the guidance information on the basis of the position that is estimated by the estimation means.

5. The image processing apparatus according to one of claims 1 to 3, wherein the generation means generates a movement distance and a movement direction of the probe as the guidance information.

6. The image processing apparatus according to one of claims 1 to 4, wherein
the predetermined structure portion includes a plurality of structure portions, and
the generation means generates the guidance information relating to each of the plurality of structure portions.

7. The image processing apparatus according to claim 6, wherein
the generation means generates the guidance information such that a structure portion of the plurality of structure portions that is located at a position closest to the imaging range of the image is included within the imaging range.

8. The image processing apparatus according to claim 2 or 4, wherein
the predetermined structure portion includes a plurality of structure portions, and
the generation means generates the guidance information for a structure portion of the plurality of structure portions for which estimation of the information is not completed.

9. The image processing apparatus according to one of claims 1 to 8, wherein
the generation means inputs the image to a trained model, and acquires the guidance information output from the trained model.

10. The image processing apparatus according to claim 2 or 4, wherein
the generation means inputs the image to a trained model, and acquires information relating to the predetermined structure portion output from the trained model.

11. The image processing apparatus according to claim 10, wherein
training data used for training of the trained model includes a set of a training image including a range larger than a range obtained by a single imaging operation using the probe, and information relating to a position and orientation of the predetermined structure portion in the training image.

12. The image processing apparatus according to claim 11, wherein
the training image is generated by combining a plurality of images in which a part of the subject is captured.

13. The image processing apparatus according to claim 11, wherein
the training image is generated from a series of images of the subject captured by mechanically controlling operation of the probe.

14. The image processing apparatus according to one of claims 1 to 13, further comprising:
precise estimation means configured to estimate, when the probe is guided on the basis of the guidance information and the predetermined structure portion enters an imaging range of the probe, the information relating to the predetermined structure portion on the basis of an image captured by the probe.

15. The image processing apparatus according to one of claims 2 to 4, wherein
the image is a three-dimensional volume image,
the estimation means estimates a position and orientation of at least one predetermined cross section of the subject located outside of the three-dimensional volume on the basis of the three-dimensional volume image,
the generation means generates guidance information for guiding a position and orientation of the probe on the basis of an estimation result of the position and orientation of the predetermined cross section obtained by the estimation means,
the image processing apparatus further comprises precise estimation means configured to estimate, when the probe is guided on the basis of the guidance information and the predetermined cross section enters an imaging range of the probe, the position and orientation of the predetermined cross section on the basis of an image captured by the probe, and
the generation means generates, when a displacement amount regarding the estimated position and orientation of the predetermined cross section with respect to a position of the three-dimensional volume being greater than a predetermined value, the guidance information such that the predetermined cross section is included within the three-dimensional volume, and generates, when the displacement amount being less than or equal to the predetermined value, the guidance information such that the position and orientation of the predetermined cross section satisfy a predetermined imaging condition of the predetermined cross section by the probe in the three-dimensional volume.

16. The image processing apparatus according to one of claims 1 to 15, wherein
the acquisition means sequentially acquires images representing a part of the subject, and
the generation means sequentially updates the guidance information on the basis of the sequentially acquired images.

17. The image processing apparatus according to claim 14, wherein
the estimation means integrates estimation results obtained during the guidance to improve an expectation value of accuracy of an estimation result by the estimation means.

18. The image processing apparatus according to claim 14, wherein
the estimation means changes an image region of a resolution used for estimation on the basis of a progress status of the guidance to improve an expectation value of accuracy of an estimation result.

19. The image processing apparatus according to claim 14, further comprising guidance status presentation means configured to present information relating to a progress status of the guidance.

20. The image processing apparatus according to claim 14, further comprising estimation status presentation means configured to present information relating to a progress status of estimation by the precise estimation means.

21. A control method of an image processing apparatus, the control method comprising:
an acquisition step of acquiring an image that represents a part of a subject and is captured using a probe; and
a generation step of generating, on the basis of the image, guidance information for guiding the probe such that a predetermined structure portion of the subject located outside of an imaging range of the image is included within the imaging range.

22. A computer program comprising instructions which, when the program is executed by a computer, cause the computer to carry out the control method of claim 21.
